# EUROPEAN PATENT APPLICATION

(11) **EP 3 156 501 A1**
(43) Date of publication of application: **19.04.2017**
(21) Application number: 15806553.2
(22) Date of filing: 11.06.2015
(51) Int. Cl.: C12Q 1/68, C12N 15/09, G01N 33/53, G01N 33/569

(54) **METHOD FOR DETECTING ESCHERICHIA COLI AND CARRIER FOR DETECTING ESCHERICHIA COLI**

(30) Priority: 11.06.2014 JP 2014120916
(71) Applicant: Toyo Seikan Group Holdings, Ltd., Shinagawa-ku Tokyo 141-8627 (JP)
(72) Inventor: NAKAJIMA, Kazuki, Yokohama-shi Kanagawa 240-0062 (JP); YAMASAKI, Takaaki, Yokohama-shi Kanagawa 240-0062 (JP); FURUKAWA, Satoshi, Yokohama-shi Kanagawa 240-0062 (JP)
(74) Representative: Pestalozzi, Deborah
(86) International application number: PCT/JP2015/002934
(87) International publication number: WO 2015/190109

(57) **Abstract**

To enable simultaneous detection with a high specificity of Escherichia *coli* including those having pathogenicity and having no pathogenicity and enterohemorrhagic *Escherichia coli* that causes food poisoning. Provided is a method for simultaneously detecting enterohemorrhagic *Escherichia coli* and *Escherichia coli* (including enterohemorrhagic *Escherichia coli*)*,* wherein genomic DNA extracted from a sample is used as a template, DNA fragments comprising a uridine monophosphate kinase gene (pyrH), DNA fragments comprising a verotoxin 1 gene (vtx1) and DNA fragments comprising a verotoxin 2 gene (vtx2) of *Escherichia coli* are amplified by a multiplex PCR, and presence or absence of each of the amplified products is detected, whereby presence or absence of enterohemorrhagic *Escherichia coli* and *Escherichia coli* in a sample is simultaneously detected with a high specificity.

## Description

### TECHNICAL FIELD

The present invention relates to a technology for detecting microorganisms such as food poisoning bacteria. In particular, the present invention relates to a method for detecting *Escherichia coli* and a carrier for detecting *Escherichia coli.*

### BACKGROUND ART

In recent years, due to improvement in health level in the field of public health such as food and environment, occurrence of food poisoning tends to be decreased. However, at present, 20,000 or more people are infected with food poisoning in Japan every year. Not a few of these food poisoning are caused by pathogenic *Escherichia coli* such as enterohemorragic *Escherichia coli* In order to prevent occurrence of food poisoning by *Escherichia coli,* in inspection of food poisoning bacteria or the like, it has become important to detect pathogenic *Escherichia coli* in food or environment with a high accuracy.

As for the method for detecting pathogenic *Escherichia coli,* as stated in Patent Documents 1 and 2, a method is known in which a primer that can amplify a verotoxin gene (Vero) is used and a DNA fragment containing the gene is amplified by a PCR (polymerase chain reaction) method and the size of an amplified product is analyzed by an electrophoresis method. In addition, as in the case of the invention disclosed in Patent Document 3 made by the applicant of the present application, a method can be given in which detection of pathogenic *Escherichia coli* is conducted by using a DNA chip to which a probe that is complementarily bonded to an amplified product of PCR is immobilized.

### RELATED ART DOCUMENTS

### PATENT DOCUMENTS

Patent Document 1: Patent No. 2905945
Patent Document 2: Patent No. 2775663
Patent Document 3: WO2011/142119

### SUMMARY OF THE INVENTION

### PROBLEMS TO BE SOLVED BY THE INVENTION

Meanwhile, in the field of public health, not only inspection of presence or absence of enterohemorragic *Escherichia coli* causing food poisoning, as the index of hygiene such as fecal contamination, it is desired that presence or absence of *Escherichia coli* be inspected simultaneously irrespective of pathogenicity.

However, by the above-mentioned conventional technology, it was impossible to detect *Escherichia coli* including those having no pathogenicity simultaneously with pathogenic *Escherichia coli.*

Further, in an actual site where inspection of *Escherichia coli* is conducted, since various DNAs such as genes of foods are mixed in an inspection sample, detection accuracy improved in specificity has been demanded.

The present invention has been made taking the above-mentioned circumstances into consideration, and is aimed at providing a method for detecting *Escherichia coli* and a carrier for detecting *Escherichia coli* that can conduct detection of *Escherichia coli* including those having no pathogenicity and enterohemorrhagic *Escherichia coli* causing food poisoning simultaneously with a high specificity.

### MEANS FOR SOLVING THE PROBLEMS

In order to attain the above-mentioned object, the method for detecting *Escherichia coli* according to the present invention is a method for detecting *Escherichia coli* that detects enterohemorrhagic *Escherichia coli* and *Escherichia coli* (including enterohemorrhagic *Escherichia coli*) simultaneously, wherein
genomic DNA extracted from a sample is used as a template, DNA fragments comprising a uridine monophosphate kinase gene (pyrH) of *Escherichia coli,* DNA fragments comprising a verotoxin 1 gene (vtx1) and DNA fragments comprising a verotoxin 2 gene (vtx2) are amplified simultaneously by a multiplex PCR, and presence or absence of each amplified product is detected, whereby presence or absence of enterohemorrhagic *Escherichia coli* and *Escherichia coli* in a sample is simultaneously detected.

Further, the carrier for detecting *Escherichia coli* according to the present invention is a carrier for detecting *Escherichia coli* for simultaneously detecting enterohemorrhagic *Escherichia coli* and *Escherichia coli* (including enterohemorrhagic *Escherichia coli*)*,* wherein
at least two or more probes composed of base sequences represented by SEQ. ID. Nos: 7 to 9 selected from a uridine monophosphate kinase gene (pyrH) of *Escherichia coli*;
at least any probe composed of base sequences represented by SEQ ID Nos:. 10 to 17 selected from a verotoxin 1 gene (vtx1) of *Escherichia coli*; and
at least any probe composed of base sequences represented by SEQ ID Nos:. 18 to 22 selected from a verotoxin 2 gene (vtx2) of *Escherichia coli*;
are immobilized.

### ADVANTAGEOUS EFFECTS OF THE INVENTION

According to the present invention, it is possible to conduct detection of *Escherichia coli* including those having pathogenicity and having no pathogenicity and enterohemorrhagic *Escherichia coli* that cause food poisoning simultaneously with a high specificity.

### BRIEF DESCRIPTION OF THE DRAWINGS

Fig. 1 is a view showing the verotoxin gene possession state of bacterial strains in the Experiments 1 and 3 in the method for detecting *Escherichia coli* and the carrier for detecting *Escherichia coli* according to the embodiment of the present invention;
Fig. 2 is a view showing a base sequence (primer sequence) of three primer sets used in the method for detecting *Escherichia coli* according to the embodiment of the present invention;
Fig. 3 is a view showing the results of an electrophoresis of an amplified product by a multiplex PCR in the method for detecting *Escherichia coli* according to the embodiment of the present invention;
Fig. 4 is a view showing the results of an electrophoresis of PCR amplified products of determination target species obtained by using a primer set (pyrH primer set) for amplifying an uridine monophosphate kinase gene region in the method for detecting *Escherichia coli* according to the embodiment of the present invention;
Fig. 5 is a view showing the results of detection (fluorescent intensity, S/N ratio) of determination several bacterial species by each probe (pyrH probe) selected from an uridine monophosphate kinase gene region in the method for detecting *Escherichia coli* and the carrier for *Escherichia coli* according to the embodiment of the present invention;
Fig. 6 is a view showing a base sequence of a probe (probe sequence) of the carrier for *Escherichia coli* according to the embodiment of the present invention; and
Fig. 7 is a view showing the results of detection (fluorescent intensity) by each probe in the method for detecting *Escherichia coli* and the carrier for *Escherichia coli* according to the embodiment of the present invention.

### MODE FOR CARRYING OUT THE INVENTION

Hereinbelow, a detailed explanation will be made on the method for detecting *Escherichia coli* and the carrier for *Escherichia coli* according to the embodiment of the present invention.

The method for detecting *Escherichia coli* according to the present embodiment is a method for detecting *Escherichia coli* that detects enterohemorrhagic *Escherichia coli* and *Escherichia coli* (including enterohemorrhagic *Escherichia coli*) simultaneously, wherein
genomic DNA extracted from a sample is used as a template, DNA fragments comprising a uridine monophosphate kinase gene (pyrH) of *Escherichia coli,* DNA fragments comprising a verotoxin 1 gene (vtx1) and DNA fragments comprising a verotoxin 2 gene (vtx2) are amplified by a multiplex PCR simultaneously, and presence or absence of each amplified product is detected, whereby presence or absence of enterohemorrhagic *Escherichia coli* and *Escherichia coli* in a sample is simultaneously detected.

*Escherichia coli* is a gram-negative facultative anaerobic bacillus and is one of intestinal bacteria. Many of them do not have pathogenicity and are harmless to human beings. However, in the field of public health, as the index for hygiene, irrespective of presence or absence of pathogenicity, it is preferred that a wide variety of *Escherichia coli* be detectable.

In the method for detecting *Escherichia coli* according to the present embodiment, an uridine monophosphate kinase gene (pyrH) that is commonly possessed by genomic DNA of *Escherichia coli* is amplified as a region to be amplified (gene region to be amplified, target gene region) by a PCR method, and an amplified product is detected. As a result, presence or absence of *Escherichia coli* including those having pathogenicity and having no pathogenicity in a sample can be detected.

Some types of *Escherichia coli* cause stomachache, diarrhea or the like. These types of *Escherichia coli* are generally called pathogenic *Escherichia coli.* In particular, some of enterohemorragic *Escherichia coli* (EHEC), representative examples of which are known as 0157, have verotoxin 1 gene (vtx1) and/or verotoxin 2 gene (vtx2).

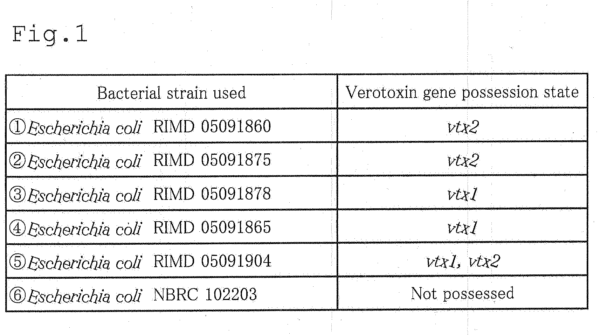

Specifically, as shown in Fig. 1, for example, there are some bacterial strains for which the presence of a verotoxin gene is known. In the following examples, by using these bacterial strains, three genes, i.e. pyrH, vtx1 and vtx2, are simultaneously detected.

In the method for detecting *Escherichia coli* according to the present embodiment, the above-mentioned uridine monophosphate kinase gene (pyrH) and verotoxin genes (vtx1, vtx2) are simultaneously amplified by a PCR method as target regions for amplification. By detecting an amplified product thereof, presence or absence of *Escherichia coli* including enterohemorragic *Escherichia coli* and presence or absence of enterohemorragic *Escherichia coli* in a sample can be simultaneously detectable.

As a result, by the method for detecting *Escherichia coli* according to the present embodiment, as for *Escherichia coli,* simultaneous detection of two indices, i.e. health indicator bacteria and food poisoning causative bacteria that could not be attained by conventional methods can become possible.

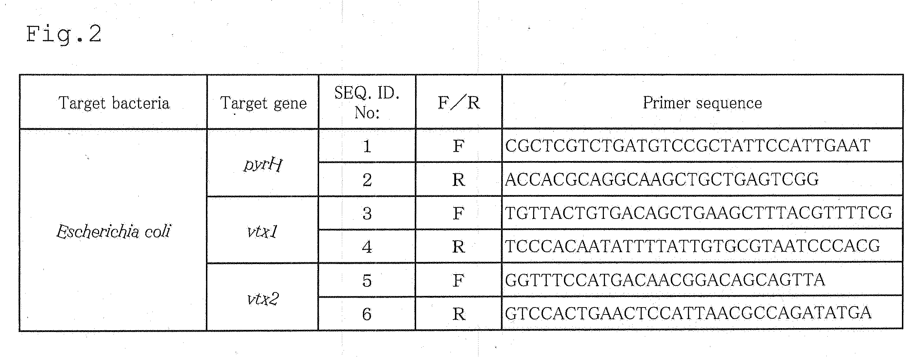

In the method for detecting *Escherichia coli* according to the present embodiment, as shown in Fig. 2, as a primer set (pyrH primer set) for amplifying an uridine monophosphate kinase gene (pyrH) region of enterohemorragic *Escherichia coli* by a PCR method, it is preferable to use a primer set comprising a primer composed of a base sequence represented by SEQ. ID. No. 1 (F: forward primer) and a primer composed of a base sequence represented by SEQ. ID. No. 2 (R: reverse primer). As a primer set (vtx1 primer set) for amplifying the verotoxin 1 gene (vtx1) region of enterohemorragic *Escherichia coli* by a PCR method, it is preferable to use a primer set comprising a primer composed of a base sequence represented by SEQ. ID. No: 3 (F: forward primer) and a primer composed of a base sequence represented by SEQ. ID. No: 4 (R: reverse primer).

Further, as a primer set (vtx2 primer set) for amplifying a verotoxin 2 gene (vtx2) region of enterohemorragic *Escherichia coli* by a PCR method it is preferable to use a primer set comprising a primer composed of a base sequence represented by SEQ ID No:. 5 (F: forward primer) and a primer composed of a base sequence represented by SEQ ID No:. 6 (R: reverse primer).

In the method for detecting *Escherichia coli* according to the present embodiment, the PCR reaction liquid comprises the above-mentioned pyrH primer set, the vtx1 primer set and the vtx2 primer set, and as for other components, common components can be used. Specifically, those containing a buffer solution, a nucleic acid substrate, a nucleic acid polymerase such as Ex Taq, labeling components such as Cy5, DNA of a sample, water or the like can be used.

By using such PCR reaction solutions and by means of a nucleic acid amplification apparatus such as a thermal cycler, part of genomic DNA in a sample is amplified. That is, when genomic DNA having a target region for amplification by the primer set is present in a sample, the target region thereof is amplified.

The primer used in the method for detecting *Escherichia coli* according to the present embodiment is not limited to those having the above-mentioned base sequence. It is also possible to use a primer in which, in each base sequence, one or several bases are deleted, substituted or added.

Then, it is preferable to detect presence or absence of *Escherichia coli* or enterohemorragic *Escherichia coli* in a sample by confirming whether an amplified product is obtained by each of the above-mentioned primer sets in the present embodiment by conducting an electrophoresis, for example, by using an amplified product (PCR amplified product) obtained by a PCR method. The electrophoresis can be conducted by a common method such as agarose gel electrophoresis, polyacrylamide gel electrophoresis and microchip electrophoresis.

It is also preferable to detect presence or absence of *Escherichia coli* or enterohemorragic *Escherichia coli* in a sample by adding dropwise a PCR amplified product to a carrier (DNA chip) for detecting *Escherichia coli* according to the present embodiment, and by detecting the label of an amplified product hybridized with the probe immobilized to the carrier.

Detection of a label can be conducted by using a common label detector such as a fluorescent scanning apparatus. For example, by using BIOSHOT (R) manufactured by Toyo Kohan Co., Ltd., detection can be conducted by measuring a fluorescent intensity of an amplified product. As the measurement results, in addition to the fluorescent intensity, it is preferable to calculate an S/N ratio (Signal to Noise ratio, (median fluorescent intensity value - background value) ÷ background value). The reason is that whether the measurement results are positive or negative can be judged with a high accuracy based on the S/N value. In general, if the S/N value is 3 or more, the measurement results are judged to be positive. The label is not restricted to fluorescence, and other labels may be used.

The carrier for detecting *Escherichia coli* according to the present embodiment is preferably one in which, as the probe (pyrH probe) selected from an uridine monophosphate kinase gene (pyrH) region of *Escherichia coli,* at least any probe composed of base sequences represented by SEQ. ID. Nos: 7 to 9 is immobilized. Further, in respect of specificity, the carrier is preferably one in which at least any probe composed of a base sequence represented by SEQ. ID. No: 8 or SEQ. ID. No: 9 is immobilized. It is further preferable that the carrier be one in which at least two or more probes composed of base sequences represented by SEQ. ID. Nos: 7 to 9 are immobilized. As mentioned above, by combining probes which have excellent specificity, it becomes possible to lower the possibility of making a false positive judgment.

Further, the carrier for detecting *Escherichia coli* according to the present embodiment is preferably one in which, as the probe (vtx1 probe) selected from the verotoxin 1 gene (vtx1) region of enterohemorragic *Escherichia coli,* at least any probe composed of a base sequence represented by SEQ ID Nos: 10 to 17 is immobilized, and one in which, as a probe (vtx2 probe) selected from the verotoxin 2 gene (vtx2) region of enterohemorragic *Escherichia coli,* at least any probe composed of a base sequence represented by SEQ. ID. Nos: 18 to 22 is immobilized.

By immobilizing, not only the pyrH probe, the vtx1 probe and the vtx2 probe mentioned above to the carrier for detecting *Escherichia coli* according to the present embodiment, together with *Escherichia coli* (including enterohemorragic *Escherichia coli*)*,* enterohemorragic *Escherichia coli* having the verotoxin 1 gene and/or verotoxin 2 gene can be detected specifically and simultaneously.

The probe used in the carrier for detecting *Escherichia coli* in the present embodiment is not restricted to the above-mentioned base sequence. A probe in which, in each base sequence, one or several bases are deleted, substituted or added can be used. Further, one composed of nucleic acid fragments that can be hybridized with, under stringent conditions, nucleic acid fragments composed of base sequences complementary to each base sequence can also be used. Further, a probe having a base sequence complementary to the base sequence of such probes can also be used.

The stringent conditions mean conditions under which a specific hybrid is formed and a non-specific hybrid is not formed. For example, the stringent conditions mean conditions under which DNA having a high homology (90% or more, preferably 95% or more of homology) for DNA composed of base sequences represented by SEQ ID Nos: 1 to 6 are hybridized with DNA composed of base sequences complementary to DNA composed of base sequences represented by SEQ. ID. Nos: 1 to 6. Normally, it means a case where hybridizing occurs at a temperature that is lower by about 5°C to 30°C, preferably about 10°C to 25°C, than the melting temperature (Tm) of a complete hybrid. As for the stringent conditions, conditions described in J. Sambrook, et. al., Molecular Cloning, A Laboratory Manual, Second Edition, Cold Spring Harbor Laboratory Press (1989), in particular, section 11.45 "Conditions for Hybridization of Oligonucleotide Probes" or other conditions can be used.

By using such carrier for detecting *Escherichia coli,* specifically, a prescribed buffer is mixed with a PCR amplified product, and the resultant is added dropwise to the carrier. The carrier is allowed to stand at 45°C for 1 hour, and thereafter, a PCR amplified product or the like that have not been hybridized are washed away from the carrier. Then, the carrier is put in a label detection apparatus to conduct detection of a label, and as a result, it is possible to detect simultaneously whether enterohemorragic *E*. *coli* and *Escherichia coli* (including enterohemorragic *Escherichia coli*) are present or absent in a sample.

As mentioned hereinabove, according to the method for detecting *Escherichia coli* and the carrier for *Escherichia coli* according to the present embodiment, *Escherichia coli* including those having pathogenicity and having no pathogenicity and enterohemorragic *Escherichia coli* that causes food poisoning can be conducted simultaneously with a high specificity.

### EXAMPLES

### <Test 1: Verification of simultaneous amplification using 3 types of primer sets>

By using 6 types of *Escherichia coli* bacterial strains shown in Fig. 1 for which the possession state of a verotoxin gene is known, DNA is extracted from these *Escherichia coli* by a normal method. In Fig. 1, (1) to (5) show bacterial strains that were derived from those furnished by Research Institute for Microbial Disease, Osaka University and (6) shows a bacterial strain that was derived from those furnished by Biological Resource Center of National Institute of Technology and Evaluation (NBRC).

Subsequently, by using the pyrH primer set, the vtx1 primer set and the vtx2 primer set shown in Fig. 2, according to each bacterial strain, the target regions for amplification thereof are simultaneously amplified by a multiplex PCR, and a verification was made whether the resulting amplified product could be detected or not. Specifically, the verification was conducted as follows.

The bacterial strains of the above-mentioned 6 types of *Escherichia coli* were respectively inoculated to Tryptic Soy Broth (manufactured by Becton, Dickson and Company, Japan), and cultured overnight at 37°C. 1 mL of each of the culture liquid was collected, and was centrifuged at 5000 × g for 10 minutes.

Subsequently, a supernatant was disposed. To the obtained precipitates, a lysozyme solution having a concentration of 20 mg/ml (20 mM Tris-HCl, pH8.0/2 mM EDTA, 1.2% TritonX-100) was added, and a bacteriolysis treatment was conducted at 37°C for 30 minutes. Further, by using DNeasy Blood & Tissue Kit (manufactured by Quiagen Co., Ltd.), column purification was conducted, whereby a DNA extraction liquid was obtained. This DNA extraction liquid was adjusted to have a concentration of 10 pg/µL to be a sample used in PCR.

Subsequently, a PCR reaction liquid was prepared with the following composition. Synthesis of a primer was entrusted to Sigma Aldrich Co., LLC. As for other reagents, those manufactured by Takara Bio Inc. were used.

| | |
|---|---|
| • Buffer solution (10 × Ex Taq buffer) | (2.0 µl) |
| • Nucleic acid synthesis substrate (dNTP Mixture) | (1.6 µl) |
| • F primer for amplifying pyrH of *Escherichia coli* (Cy5-modified (5' terminal)) | (0.2 µl) |
| • R primer for amplifying pyrH of *Escherichia coli* | (0.2 µl) |
| • F primer for amplifying vtx1 of *Escherichia coli* | (0.2 µl) |
| • R primer for amplifying vtx1 of *Escherichia coli* (Cy5-modified (5' terminal)) | (0.2 µl) |
| • F primer for amplifying vtx2 of *Escherichia coli* | (0.2 µl) |
| • R primer for amplifying vtx2 of *Escherichia coli* (Cy5-modified (5' terminal)) | (0.2 µl) |
| • TaKaRa Ex Taq Hot Start Version | (0.2 µl) |
| • DNA of sample | (1.0 µl) |
| • Sterilized water | (14.0 µl) |
| (Total amount: 20 µl) | |

For the amplification of a gene by PCR, a thermal cycler ep gradient (manufactured by Eppendorf Japan) was used. The reaction conditions were as follows:
(1) 95°C 2 minutes
(2) 95°C 10 seconds (denaturation step)
(3) 68°C 30 seconds (annealing step)
(4) 72°C 30 seconds (extension step)
(5) 72°C 2 minutes
Steps (2) to (4) were repeated 40 cycles.

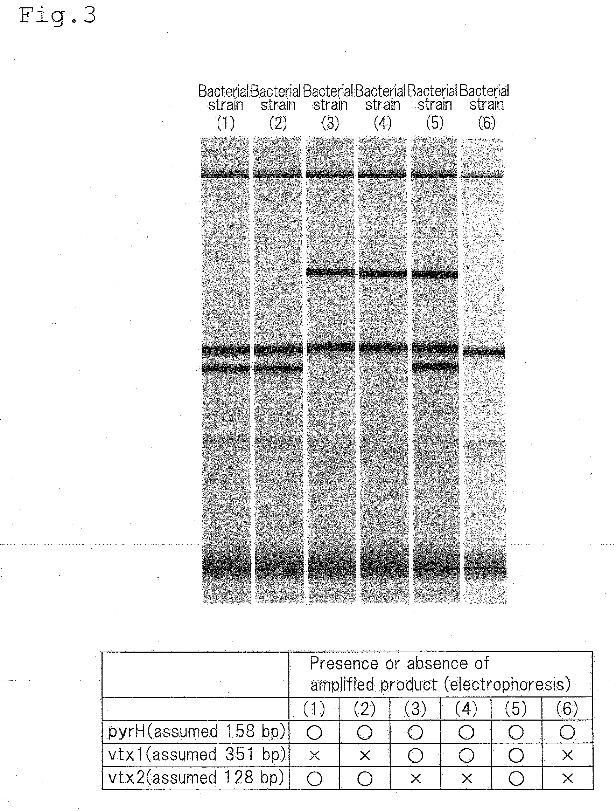

Subsequently, a PCR reaction liquid containing the thus obtained PCR amplified product was put in a microchip electrophoresis apparatus (MultiNa, manufactured by Shimadzu Corporation) and the size of a PCR amplified product was analyzed. The results are shown in Fig. 3.

As shown in Fig. 3, as a result of the electrophoresis, an amplified product by the pyrH primer set (assumed size: 158 bp) was detected for all of the 6 types of the bacterial strain.

On the other hand, the amplified product by the vtx1 primer set (assumed size: 351 bp) was detected for only the bacterial strains (3), (4) and (5) that possessed the vtx1 gene. The amplified product by the vtx2 primer set (assumed size: 128 bp) was detected for only the bacterial strains (1), (2) and (5) that possessed the vtx2 gene.

From the above, it can be understood that, by using the pyrH primer set, the vtx1 primer set and the vtx2 primer set in the method for detecting *Escherichia coli* according to the present embodiment, these regions could be detected specifically and simultaneously. As mentioned above, by the method for detecting *Escherichia coli* according to the present embodiment, it can be confirmed that detection of *Escherichia coli* and enterohemorrhagic *Escherichia coli* can be conducted simultaneously.

### <Test 2: Verification of pyrH probe of Escherichia coli>

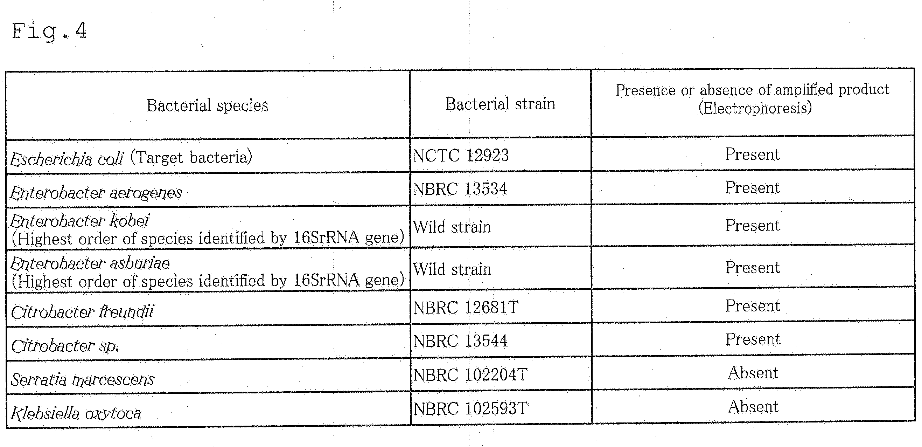

In the case where PCR is conducted by using a primer set (SEQ. ID. Nos: 1 and 2) for amplifying the pyrH region of *Escherichia coli,* when part of bacterial species such as *Enterobacter* or *Citrobacter* that are allied species of *Escherichia coli* are contained in a sample, as shown in Fig. 4, an amplified product based on genomic DNA of the bacterial species thereof can be obtained.

In such a case, when judgement of presence or absence of *Escherichia coli* and enterohemorrhagic *Escherichia coli* is conducted by electrophoresis, there is a possibility that false positive judgment results are obtained since *Escherichia coli* and enterohemorrhagic *Escherichia coli* cannot be distinguished from the allied species thereof.

Therefore, a verification was conducted whether *Escherichia coli* and these allied species can be distinguished by using the carrier for detecting *Escherichia coli* according to the present embodiment. Specifically, the following verification is conducted.

The target regions for amplification of bacterial strains of the determination target species shown in Fig. 4 were amplified according to the bacterial strain by using 10 ng/µL of a DNA extraction liquid obtained by culturing in the same manner as in Test 1 and the pyrH primer set shown in Fig. 2. In this test, a PCR reaction solution was prepared by the following method, and as for the other points, in the same manner as in test 1, a PCR reaction liquid including a PCR amplified product was obtained.

| | |
|---|---|
| • Buffer solution (10 × Ex Taq buffer) | (2.0 µl) |
| • Nucleic acid synthesis substrate (dNTP Mixture) | (1.6 µl) |
| • F primer for amplifying pyrH of *Escherichia coli* (Cy5-modified (5'-terminal)) | (0.2 µl) |
| • R primer for amplifying pyrH of *Escherichia coli* | (0.2 µl) |
| • TaKaRa Ex Taq Hot Start Version | (0.2 µl) |
| • DNA of sample | (1.0 µl) |
| • Sterilized water | (14.8 µl) |
| (Total amount: 20 µl) | |

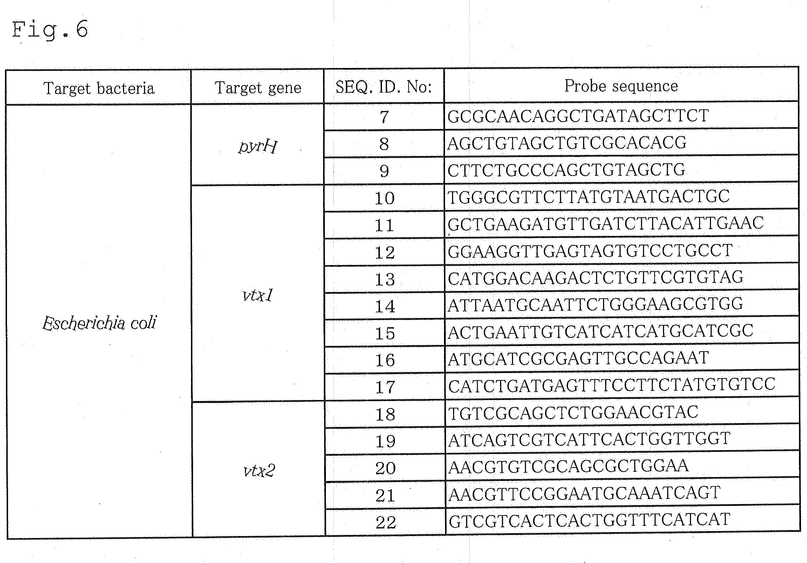

In addition, a DNA chip to which each probe composed of a base sequence represented by SEQ ID Nos: 7 to 9 shown in Fig. 6 was immobilized in advance was prepared. Then, according to the bacterial strain, a mixture obtained by mixing 4 µL of a PCR reaction liquid and 2 µLof a buffer solution for hybridization (3 × SSC/0.3%SDS citric acid-physiological saline-sodium dodecyl sulfate) was added dropwise to the above-mentioned DNA chip, and the resultant was allowed to react at 45°C for 1 hour.

After the reaction, the DNA chip was immersed in a washing liquid (2 × SSC/0.2% SDS solution, 2 × SSC solution in this order) to conduct washing at room temperature, then, the cover glass was put thereon and the fluorescence of the spot region of each probe was detected by means of a fluorescent detector BIOSHOT (manufactured by Toyo Kohan Co., Ltd.).

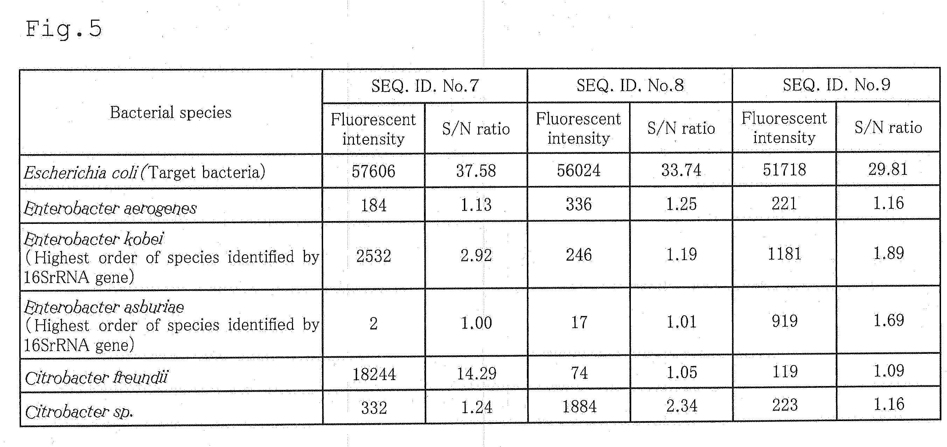

Specifically, the labeling components (Cy5) of an amplified product hybridized with the probe was excited by laser beam to emit light, and the amount of emitted light was detected by means of a CCD camera attached to the detector. Further, the amount of light was converted to electric signals to obtain numerical values, whereby a fluorescent intensity was obtained. This fluorescent intensity is an intensity index of the apparatus. No unit is used to indicate this intensity, and this intensity is calculated after correcting so that the back ground numerical value became 0. Also, an S/N ratio was calculated. The results are shown in Fig. 5.

As shown in Fig. 5, a probe composed of a base sequence represented by the SEQ. ID. No: 7 shows a high fluorescent intensity for *Escherichia coli* that is a bacterium to be detected. It also shows a relatively high fluorescent intensity for *Enterobacter kobei* and *Citrobacter freundii* that are not target bacterial species to be detected. In particular, for *Citrobacter freundii,* the S/N ratio thereof is 3 or more, and false positive reactions occur.

The probe composed of a base sequence represented by SEQ. ID. No: 8 shows a high fluorescent intensity for *Escherichia coli* that is a bacterium to be detected. It also shows a relatively high fluorescent intensity for *Citrobacter sp.* that is not target bacterial species to be detected. The S/N ratio thereof is less than 3, and no false positive reactions occur.

Further, the probe composed of a base sequence represented by SEQ. ID. No: 9 shows a high fluorescent intensity for *Escherichia coli* that is a bacterium to be detected. It also shows a relatively high fluorescent intensity for *Enterobacter kobei* that is not target bacterial species to be detected. The S/N ratio thereof is less than 3, and no false positive reactions occur.

Therefore, the carrier for detecting *Escherichia coli* according to the present embodiment is preferably one to which at least any probes composed of a base sequence represented by SEQ. ID. No: 8 or 9 are immobilized.

Further, since probes composed of a base sequence represented by SEQ. ID. Nos: 7 to 9 respectively show a high fluorescent intensity for different bacteria, by combining these, it is possible to obtain effects of suppressing occurrence of false positive judgment. Therefore, it is more preferred that the carrier for detecting *Escherichia coli* according to the present embodiment be one to which at least two or more probes composed of a base sequence represented by SEQ. ID. Nos: 7 to 9 are immobilized. It is further preferred that the carrier be one to which all of the probes composed of a base sequence represented by SEQ. ID. Nos: 7 to 9 are immobilized.

### <Test 3: Verification of simultaneous detection by DNA chip>

By using the carrier for detecting *Escherichia coli* according to the present embodiment, a verification was made whether three genes, i.e. pyrH, vtx1 and vtx2 in *Escherichia coli,* can be detected specifically and simultaneously.

Specifically, a DNA chip to which each probe composed of a base sequence represented by SEQ. ID. Nos: 7 to 22 shown in Fig. 6 was immobilized was prepared.

Then, by using 6 types of bacterial strain of *Escherichia coli* shown in Fig. 1, according to the bacterial strain, a mixture obtained by mixing 4 µL of a PCR reaction liquid obtained in the same manner as in Experiment 1 and 2 µLof a buffer solution for hybridization (3 × SSC/0.3%SDS citric acid-physiological saline-sodium dodecyl sulfate) was added dropwise to this DNA chip, and the resultant was allowed to react at 45°C for 1 hour.

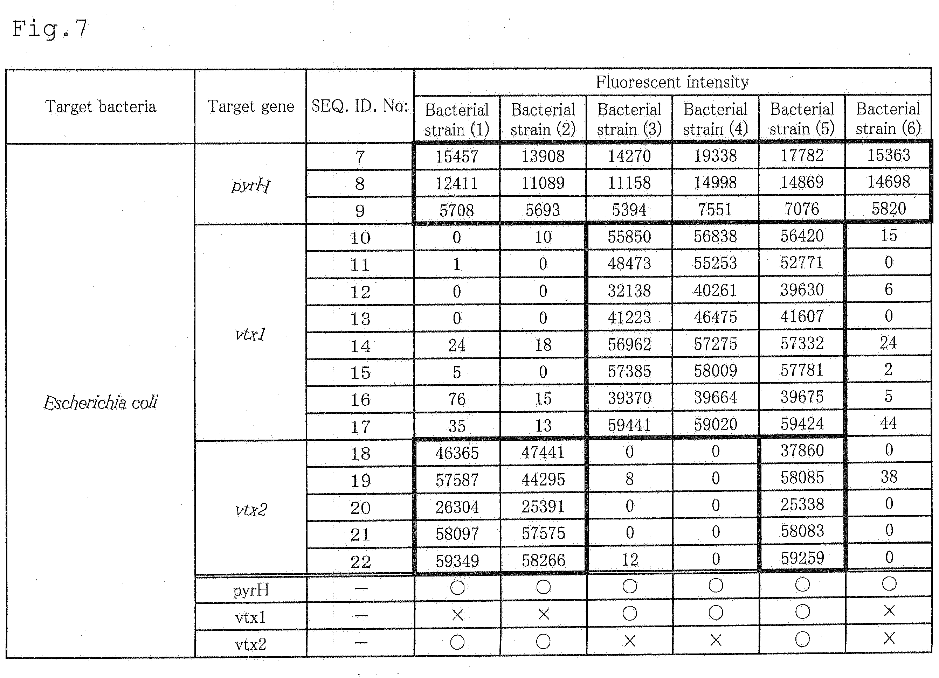

After the reaction, in the same manner as in Test 2, the DNA chip was immersed in a washing liquid (2 × SSC/0.2% SDS solution, 2 × SSC solution in this order) to conduct washing, then, the cover glass was put thereon and the fluorescence of the spot region of each probe was detected by means of a fluorescent detector BIOSHOT (manufactured by Toyo Kohan Co., Ltd.). The results are shown in Fig. 7.

As shown in Fig.7, the probe for detecting pyrH composed of base sequences represented by SEQ. ID. Nos: 7 to 9 show a high fluorescent intensity for all of the 6 types of bacterial strains of *Escherichia coli.*

On the other hand, the probe for detecting vtx1 composed of base sequences represented by SEQ. ID. Nos: 10 to 17 show a high fluorescent intensity only for bacterial strains (3), (4) and (5) that possess a vtx1 gene, and does not show a high fluorescent intensity for bacterial strains (1), (2) and (6) that do not possess a vtx1 gene. Further, the probe for detecting vtx2 composed of a base sequence represented by SEQ. ID. Nos: 18 to 22 show a high fluorescent intensity only for bacterial strains (1), (2) and (5) that possess vtx2 gene, and does not show a high fluorescent intensity for bacterial strains (3), (4) and (6) that do not possess vtx2 gene.

From the above, it has been revealed that, by the carrier for detecting *Escherichia coli* according to the present embodiment, it is possible to judge simultaneously and specifically whether DNA fragments containing three types of genes, i.e. pyrH, vtx1 and vtx2, are present or absent in a sample.

The present invention is not limited to the above-mentioned embodiments and examples, and it is possible to make various modifications within the scope the invention. For example, in the method for detecting *Escherichia coli* according to the present embodiment, it is possible to make appropriate modifications by incorporating other components into a PCR reaction liquid or by adding and immobilizing other probes than those mentioned above to the carrier for detecting *Escherichia coli* according to the present embodiment.

### INDUSTRIAL APPLICABILITY

The present invention can be preferably used when *Escherichia coli* is detected in food inspection, epidemic environmental inspection, clinical tests, environmental inspection, domestic animal health or the like.

## Claims

1. A method for detecting *Escherichia coli* that detects enterohemorrhagic *Escherichia coli* and *Escherichia coli* (including enterohemorrhagic *Escherichia coli*) simultaneously, wherein
genomic DNA extracted from a sample is used as a template, DNA fragments comprising a uridine monophosphate kinase gene (pyrH) DNA fragments comprising a verotoxin 1 gene (vtx1) and DNA fragments comprising a verotoxin 2 gene (vtx2) of *Escherichia coli,* are amplified by a multiplex PCR, and presence or absence of each amplified product is detected, whereby presence or absence of enterohemorrhagic *Escherichia coli* and *Escherichia coli* in a sample is simultaneously detected.

2. The method for detecting *Escherichia coli* according to claim 1, wherein, by using a carrier for detecting *Escherichia coli* in which a probe that is complementarily bonded with an amplified product obtained by amplifying the DNA fragments comprising pyrH, a probe that is complementarily bonded with an amplified product obtained by amplifying the DNA fragments comprising vtx1 and a probe that is complementarily bonded with an amplified product obtained by amplifying the DNA fragments comprising vtx2 are immobilized, presence or absence of each of the amplified products is simultaneously detected.

3. The method for detecting *Escherichia coli* according to claim 1 or 2, wherein, the multiplex PCR is conducted by using a PCR reaction solution comprising:
a primer set for amplifying the DNA fragments comprising pyrH comprising a primer composed of a base sequence represented by SEQ. ID. No: 1 and a primer composed of a base sequence represented by SEQ. ID. No: 2;
a primer set for amplifying the DNA fragments comprising vtx1 comprising a primer composed of a base sequence represented by SEQ. ID. No: 3 and a primer composed of a base sequence represented by SEQ. ID. No: 4; and
a primer set for amplifying the DNA fragments comprising vtx2 comprising a primer composed of a base sequence represented by SEQ. ID. No: 5 and a primer composed of a base sequence represented by SEQ. ID. No: 6, and
detection of the amplified product is conducted by using a carrier for detecting *Escherichia coli* to which at least two or more probes composed of base sequences represented by SEQ. ID. Nos: 7 to 9 selected from the pyrH;
at least any of probes composed of base sequences represented by SEQ. ID.
Nos: 10 to 17 selected from the vtx1; and
at least any of probes composed of base sequences represented by SEQ. ID. Nos: 18 to 22 selected from the vtx2
are immobilized.

4. The method for detecting *Escherichia coli* according to claim 3, wherein at least any of probes selected from each of the genes is any of the following (1) to (3):
(1) a probe in which, in a base sequence represented by the SEQ. ID. No, one or several bases are missing, substituted or added;
(2) a probe that can be hybridized under stringent conditions for nucleic acid fragments composed of a base sequence complementary to a base sequence represented by the SEQ. ID. No; and
(3) a probe having a base sequence complementary to the probe of (1) or (2).

5. A carrier for detecting *Escherichia coli* for simultaneously detecting enterohemorrhagic *Escherichia coli* and *Escherichia coli* (including enterohemorrhagic *Escherichia coli*)*,* wherein
at least two or more probes composed of base sequences represented by SEQ. ID. Nos: 7 to 9 selected from a uridine monophosphate kinase gene (pyrH) of *Escherichia coli* (pyrH);
at least any probe composed of base sequences represented by SEQ. ID. Nos: 10 to 17 selected from a verotoxin 1 gene (vtx1); and
at least any probe composed of base sequences represented by SEQ. ID. Nos: 18 to 22 selected from a verotoxin 2 gene (vtx2);
are immobilized.

6. The carrier for detecting *Escherichia coli* according to claim 5, wherein the at least any of probes selected from each of the genes is any of the following (1) to (3):
(1) a probe in which, in a base sequence represented by the SEQ. ID. No., one or several bases are missing, substituted or added;
(2) a probe that can be hybridized under stringent conditions for nucleic acid fragments comprising a base sequence complementary to each base sequence; and
(3) a probe having a base sequence complementary to the base sequence of the probe (1) or (2).


SEQUENCE LISTING

<110>  TOYO SEIKAN GROUP HOLDINGS, LTD.

<120>  METHOD FOR DETECTING ESCHERICHIA COLI AND CARRIER FOR DETECTING
       ESCHERICHIA COLI

<130>  TSK-1504-PCT

<160>  22

<170>  PatentIn version 3.5

<210>  1
<211>  31
<212>  DNA
<213>  Escherichia coli

<400>  1
cgctcgtctg atgtccgcta ttccattgaa t                                    31


<210>  2
<211>  26
<212>  DNA
<213>  Escherichia coli

<400>  2
accacgcagg caagctgctg agtcgg                                          26


<210>  3
<211>  33
<212>  DNA
<213>  Escherichia coli

<400>  3
tgttactgtg acagctgaag ctttacgttt tcg                                  33


<210>  4
<211>  32
<212>  DNA
<213>  Escherichia coli

<400>  4
tcccacaata ttttattgtg cgtaatccca cg                                   32


<210>  5
<211>  27
<212>  DNA
<213>  Escherichia coli

<400>  5
ggtttccatg acaacggaca gcagtta                                         27


<210>  6
<211>  31
<212>  DNA
<213>  Escherichia coli

<400>  6
gtccactgaa ctccattaac gccagatatg a                                    31

<210>  7
<211>  22
<212>  DNA
<213>  Escherichia coli

<400>  7
gcgcaacagg ctgatagctt ct                                              22

<210>  8
<211>  20
<212>  DNA
<213>  Escherichia coli

<400>  8
agctgtagct gtcgcacacg                                                 20

<210>  9
<211>  20
<212>  DNA
<213>  Escherichia coli

<400>  9
cttctgccca gctgtagctg                                                 20

<210>  10
<211>  24
<212>  DNA
<213>  Escherichia coli

<400>  10
tgggcgttct tatgtaatga ctgc                                            24

<210>  11
<211>  27
<212>  DNA
<213>  Escherichia coli

<400>  11
gctgaagatg ttgatcttac attgaac                                         27

<210>  12
<211>  24
<212>  DNA
<213>  Escherichia coli

<400>  12
ggaaggttga gtagtgtcct gcct                                            24

<210>  13
<211>  25
<212>  DNA
<213>  Escherichia coli

<400>  13
catggacaag actctgttcg tgtag                                              25


<210>  14
<211>  25
<212>  DNA
<213>  Escherichia coli

<400>  14
attaatgcaa ttctgggaag cgtgg                                              25


<210>  15
<211>  26
<212>  DNA
<213>  Escherichia coli

<400>  15
actgaattgt catcatcatg catcgc                                             26


<210>  16
<211>  22
<212>  DNA
<213>  Escherichia coli

<400>  16
atgcatcgcg agttgccaga at                                                 22


<210>  17
<211>  28
<212>  DNA
<213>  Escherichia coli

<400>  17
catctgatga gtttccttct atgtgtcc                                           28


<210>  18
<211>  21
<212>  DNA
<213>  Escherichia coli

<400>  18
tgtcgcagct ctggaacgta c                                                  21


<210>  19
<211>  24
<212>  DNA
<213>  Escherichia coli

<400>  19
atcagtcgtc attcactggt tggt                                               24


<210>  20
<211>  20
<212>  DNA
<213>  Escherichia coli

<400>  20
aacgtgtcgc agcgctggaa                                                   20

<210>  21
<211>  23
<212>  DNA
<213>  Escherichia coli

<400>  21
aacgttccgg aatgcaaatc agt                                               23

<210>  22
<211>  24
<212>  DNA
<213>  Escherichia coli

<400>  22
gtcgtcactc actggtttca tcat                                              24